# EUROPEAN PATENT APPLICATION

(11) **EP 3 501 331 A1**
(43) Date of publication of application: **26.06.2019**
(21) Application number: 17841401.7
(22) Date of filing: 04.08.2017
(51) Int. Cl.: A45D 44/22

(54) **COSMETIC EYELID SHAPING TAPE**

(30) Priority: 17.08.2016 JP 2016160189
(71) Applicant: Artsbrains. Co. Ltd., Tokyo 150-0001 (JP)
(72) Inventor: NOJIRI, Hideyuki, Tokyo 151-0061 (JP)
(74) Representative: Carpmael, Robert Maurice Charles
(86) International application number: PCT/JP2017/028409
(87) International publication number: WO 2018/034176

(57) **Abstract**

[Object] To improve adhesiveness of a cosmetic eyelid shaping tape for performing pseudo plastic surgery on an eyelid at inner-canthus and outer-canthus end portions of the eyelid by forming a constricted portion in the eyelid using resilient elasticity after extension of a base piece, constituting a tape-shaped member.

[Solution] A double-eyelid-forming tape 1 includes a tape-shaped member 2 formed by laminating adhesive layers 4 on both surfaces of a base piece 3, and a pair of release sheet pieces 5 bonded to the adhesive layers. The release sheet pieces 5 each have groove-shaped tearable portions 6 and 7 that are easily tearable when being pulled in a longitudinal direction. The tearable portions each have a groove width gradually expanding from a groove bottom 61 or 71 toward the outer surface of the corresponding release sheet piece. The tearable portions include first tearable portions 6 formed at positions on the sides of a center O of the tape 1 closer to a first end 1a and a second end 1b, and a second tearable portion 7 at the center O and deeper than the first tearable portion.

## Description

### Technical Field

The present invention relates to a cosmetic eyelid shaping tape. The tape includes a base piece constituting a tape-shaped member, the base piece being formed from a synthetic resin extensible into a plastic region and having resilient elasticity after being extended. The cosmetic eyelid shaping tape is used for performing pseudo plastic surgery on the eyelid, such as forming an imitation double eyelid crease in the upper eyelid using resilient elasticity of the base piece when the base piece is extended by elongating the tape-shaped member.

Double-eyelid-forming cosmetic products known thus far for forming an imitation double eyelid without surgery include solution type products (Patent Literatures 1 to 3) each including a solution to be applied to the skin of the upper eyelid, and tape type products (Patent Literatures 4 to 8) each including an adhesive tape adhering to the skin of the upper eyelid.

According to the mechanism for forming the double eyelid, these solution and tape type products are mainly classified into a method (adhesion method) for forming a double eyelid crease by bonding folded portions of the skin of the upper eyelid together with, for example, a double-sided adhesive tape or adhesive, and a method (shutter method) for forming a double eyelid crease by forming a hard coating on the skin of the upper eyelid and allowing the skin of the upper eyelid to be folded along the upper edge of the coating when the eyelid is opened. The hard coating is formed by, for example, applying a solution onto the skin of the upper eyelid and drying the solution or by attaching an adhesive tape onto the skin of the upper eyelid.

These existing double-eyelid-forming cosmetic products, however, forcibly form a double eyelid crease using adhesion or a coating with a solution or a tape. These existing products thus have various problems including: double eyelids thus formed often seem unnatural, such products are conspicuous when the upper eyelids are closed, or users are more likely to feel discomfort, such as a feeling of being pulled.

To solve the above-described problems, a double-eyelid forming tape (Patent Literature 9) for forming a double eyelid by using a mechanism completely different from the above-described existing methods has been disclosed. This double-eyelid forming tape is formed of a tape-shaped member including an adhesive applied to an extensible base piece having elastic contractility even after being extended. Specifically, the double-eyelid-forming tape is used to form a groove-shaped constricted portion in the upper eyelid along the tape-shaped member using the elastic contractility after extension of the base piece forming the tape-shaped member. When the upper eyelid is opened, the skin of the upper eyelid is thus naturally folded over along the constricted portion to form a double eyelid crease. Besides transforming a single eyelid into a double eyelid, the tape is currently used to perform a variety of make-ups (that is, pseudo plastic surgery) on the eyelid, such as to change the position, shape, or vertical width of a double eyelid crease, or to form puffiness (a so-called eye bag) at the lower eyelid.

### Citation List

### Patent Literature

PTL 1: Japanese Unexamined Patent Application Publication No. 2007-106711
PTL 2: Japanese Registered Utility Model No. 3111511
PTL 3: Japanese Unexamined Patent Application Publication No. 02-188512
PTL 4: Japanese Registered Utility Model No. 3154139
PTL 5: Japanese Unexamined Patent Application Publication No. 10-304935
PTL 6: Japanese Unexamined Patent Application Publication No. 2005-334108
PTL 7: Japanese Unexamined Patent Application Publication No. 2007-111218
PTL 8: Japanese Unexamined Patent Application Publication No. 2009-195410
PTL 9: Japanese Patent No. 3277180

### Summary of Invention

### Technical Problem

In the above-described cosmetic eyelid shaping tapes used for performing pseudo plastic surgery on an eyelid by forming a constricted portion in the eyelid using the elastic contractility after extension of the base piece forming the tape-shaped member, the tape-shaped member needs to be pressed against and bonded to the eyelid while being elongated and stretched. The eyelid skin is formed of a complex surface curved to correspond to, for example, the skeletal structure of the eyeball or the surroundings of the eyeball. The above bonding operation with pressure does not necessarily easily secure the sufficiently high adhesiveness throughout the entire portion, in the longitudinal direction, of the tape-shaped member bonded to the eyelid.

A user may thus have the portion of the tape-shaped member bonded to the eyelid more likely more likely to come off at, particularly, an inner canthus end or an outer canthus end.

A technical object of the present invention is thus to provide a cosmetic eyelid shaping tape for performing pseudo plastic surgery on an eyelid by forming a constricted portion in the eyelid, the tape including a tape-shaped member having improved adhesiveness to the skin at an inner canthus end or an outer canthus end of the eyelid, the tape-shaped member including a base piece and an adhesive layer laminated on the base piece, the base piece being formed from a synthetic resin extensive into the plastic region and having resilient elasticity even after being extended. The constricted portion is formed in the eyelid using elastic contractility of the base piece when the tape-shaped member is elongated until the base piece is extended.

### Solution to Problem

To achieve the above object, the present invention provides a cosmetic eyelid shaping tape for performing pseudo plastic surgery on an eyelid by forming a constricted portion in the eyelid. The tape includes a tape-shaped member including a long and narrow base piece having a uniform width and adhesive layers laminated on both surfaces of the base piece. The base piece is formed from a synthetic resin extensive into a plastic region and having resilient elasticity even after being extended. The constricted portion is formed in the eyelid using elastic contractility of the base piece when the tape-shaped member is elongated until the base piece is extended. The cosmetic eyelid shaping tape further includes a pair of release sheet pieces formed from a synthetic resin that is easily releasable from the adhesive layers. The release sheet pieces are bonded to cover the adhesive layers of the tape-shaped member. The release sheet pieces each have a first end and a second end on both ends in a longitudinal direction. The pair of release sheet pieces each have a tearable portion on at least one side of a longitudinal center closer to the first end and/or the second end. The tearable portion is torn when being pulled in the longitudinal direction. The tearable portion has a shape of a groove having a depth from an outer surface of each release sheet piece to a middle in a thickness direction and extending in a width direction.

In such a cosmetic eyelid shaping tape according to the present invention, when the tearable portions of the pair of release sheet pieces are torn by being pulled in the longitudinal direction and the tape-shaped member is then elongated by being pulled in the longitudinal direction, the base piece is accordingly extended into the plastic region. At this time, in the base piece having a uniform width, the extension is usually sequentially transmitted in the longitudinal direction from around the position of the torn tearable portion. In accordance with the progress of the extension, the adhesive layers are also gradually thinned. However, the transmission and the progress of the extension are slower as the distance from the torn tearable portion becomes longer. Thus, the adhesive layers having a smaller extension ratio are left thicker, and secure more efficient adhesiveness to the eyelid skin.

Thus, when the cosmetic eyelid shaping tape is to be applied to an eyelid and, for example, to improve the adhesiveness of the tape-shaped member at, for example, the outer canthus or the inner canthus, each release sheet piece is torn at a to-be-torn tearable portion while a longer side of the tape-shaped member, having a longer length from the to-be-torn tearable portion to the end of the tape-shaped member, is located on the portion of the skin intended to have improved adhesiveness. Then, the tape-shaped member is elongated to a predetermined length to extend the base piece into the plastic region. Subsequently, while being elongated to the predetermined length (that is, while being pulled tightly), the tape-shaped member is pressed against the portion (line) of the eyelid at which the constricted portion is intended to be formed, and bonded to the eyelid with the adhesive layers. Subsequently, when the tensile force exerted on the tape-shaped member and the pressing force exerted on the eyelid are unloaded, the elastic contractile force of the extended base piece is exerted on the eyelid, and the tape-shaped member (that is, the base piece) is introduced into the eyelid to form a constricted portion in the eyelid. Thus, the eyelid can undergo cosmetic blepharoplasty using the formed constricted portion of the eyelid, such as forming a double eyelid fold in an upper eyelid or forming puffiness (a so-called eye bag) at a lower eyelid. Here, the portion of the base piece having a smaller extension ratio and the thicker portions of the adhesive layers are left on the longer side of the tape-shaped member, having a longer length from the torn tearable portion to the end of the tape-shaped member. Thus, the adhesiveness to the eyelid skin can be improved.

In the cosmetic eyelid shaping tape, preferably, each tearable portion has a shape of a groove having a groove width gradually expanding from a groove bottom toward the outer surface of the release sheet piece. Here, the groove bottom of the tearable portion may be a flat surface. If a plurality of the tearable portions are formed at a regular pitch in the longitudinal direction of the release sheet pieces, the torn position of the tearable portion of each release sheet piece can be adjusted, so that the adhesiveness to the eyelid skin of the tape-shaped member when elongated to a predetermined length can be adjusted in accordance with the preference. In addition, the groove bottoms of the tearable portions adjacent to each other may be connected together with arcuate surfaces rising from the groove bottoms and having the same height.

In the cosmetic eyelid shaping tape according to the present invention, each of the release sheet pieces may have the same number of tearable portions on both sides of the longitudinal center closer to the first end and the second end. Here, each of the release sheet pieces may have the tearable portions at symmetrical positions on the sides closer to the first end and the second end with respect to the longitudinal center.

In the cosmetic eyelid shaping tape according to the present invention, preferably, the tearable portion forms a first tearable portion, and the pair of release sheet pieces each further include a second tearable portion at the longitudinal center, the second tearable portion being torn when being pulled in the longitudinal direction, and the second tearable portion has a shape of a groove having a depth from an outer surface of each release sheet piece to a middle in a thickness direction and extending in a width direction. Thus, in accordance with the preference, the base piece can be also used in a conventional manner to be extended equidistantly toward the first end and the second end of the tape-shaped member. Here, more preferably, the second tearable portion has a shape of a groove having a groove width gradually expanding from a groove bottom toward the outer surface of the release sheet piece, and having a larger depth than the first tearable portion. Thus, when each release sheet piece is intended to be torn at the second tearable portion, the release sheet piece can be more reliably torn at the tearable portion. Here, more preferably, the groove bottom of the second tearable portion forms an acute angle. Also preferably, the pair of release sheet pieces each have a plurality of the first tearable portions on both sides of the second tearable portion in the longitudinal direction closer to the first end and the second end, the first tearable portions being arranged at a regular pitch in the longitudinal direction of the release sheet piece. On an outer surface of the release sheet piece at a portion adjacent to the second tearable portion in the longitudinal direction, a flat portion not including the first tearable portion is formed. Thus, the second tearable portion can be easily distinguished from the first tearable portions.

### Advantageous Effects of Invention

As described above, the present invention can provide a cosmetic eyelid shaping tape for performing pseudo plastic surgery on an eyelid by forming a constricted portion in the eyelid, the tape including a tape-shaped member having improved adhesiveness to the skin at an inner canthus end or an outer canthus end of the eyelid, the tape-shaped member including a base piece and an adhesive layer laminated on the base piece, the base piece being formed from a synthetic resin extensive into the plastic region and having resilient elasticity even after being extended. The constricted portion is formed in the eyelid using elastic contractility of the base piece when the base piece is extended by elongating the tape-shaped member.

### Brief Description of Drawings

Fig. 1 is a front view of a double-eyelid-forming tape, which is an embodiment of a cosmetic eyelid shaping tape according to the present invention.
Fig. 2 is a plan view of the tape illustrated in Fig. 1.
Fig. 3 is a cross-sectional view of the tape illustrated in Fig. 1 taken along line A-A.
Fig. 4 is an exploded perspective view of the tape illustrated in Fig. 1.
Fig. 5 is an enlarged view of a center portion B of the tape illustrated in Fig. 1.
Fig. 6 schematically illustrates a "tensile stress-strain curve", representing general tensile properties of a "synthetic resin extensible into the plastic region and having resilient elasticity even after being extended", forming the base piece of the tape illustrated in Fig. 1.
Fig. 7 is schematic diagram 1 illustrating the method for using the double-eyelid-forming tape illustrated in Fig. 1.
Fig. 8 is schematic diagram 2 illustrating the same.
Fig. 9 is schematic diagram 3 illustrating the same.
Fig. 10 is schematic diagram 4 illustrating the same.
Fig. 11 is schematic diagram 5 illustrating the same.
Fig. 12 is schematic diagram 6 illustrating the same.
Fig. 13 is schematic diagram 7 illustrating the same.
Fig. 14 is schematic diagram 8 illustrating the same.
Fig. 15 is schematic diagram 9 illustrating the same.
Fig. 16 is a schematic diagram illustrating another method for using a cosmetic eyelid shaping tape according to the present invention.
Fig. 17 is schematic diagram 1 illustrating the procedure of manufacturing a cosmetic eyelid shaping tape according to the present invention.
Fig. 18 is schematic diagram 2 illustrating the same.
Fig. 19 is schematic diagram 3 illustrating the same.
Fig. 20 is schematic diagram 4 illustrating the same.
Fig. 21 is a front view of another embodiment of a cosmetic eyelid shaping tape according to the present invention.

### Description of Embodiments

Hereinbelow, one embodiment of a cosmetic eyelid shaping tape according to the present invention will be described below in detail with reference to the drawings. In the following description, a double-eyelid-forming tape is mainly taken as an example of the cosmetic eyelid shaping tape.

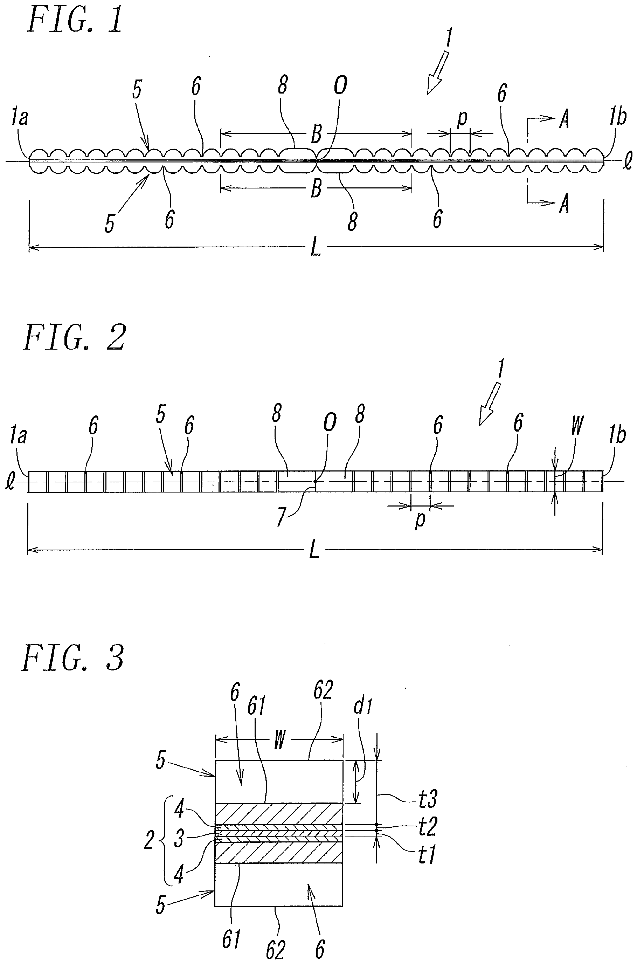

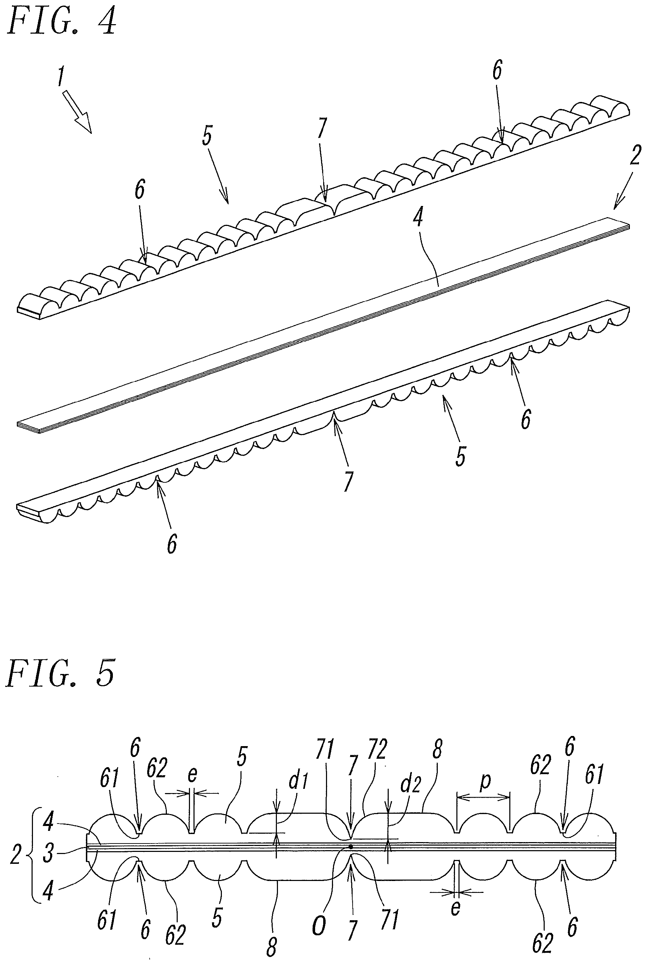

As illustrated in Fig. 1 to Fig. 5, a double-eyelid-forming tape 1, serving as a cosmetic eyelid shaping tape according to the present invention, includes a long, narrow film-shaped base piece 3 formed from a synthetic resin extensible into the plastic region and having resilient elasticity even after being extended, and adhesive layers 4 laminated on the base piece 3 with an adhesive applied to both entire surfaces of the base piece 3 to be bonded to the upper eyelid. A double eyelid fold is formed in the upper eyelid using elastic contractility after extension of the base piece 3 when a tape-shaped member 2 is elongated in the direction of a longitudinal axis 1 until the base piece 3 is extended.

Here, the tape-shaped member 2, that is, the base piece 3 constituting the tape-shaped member 2 forms a straight line in the direction of the longitudinal axis 1, and has a length L in the direction of the axis 1, and a uniform width W throughout in the direction of the axis 1. Thus, the base piece 3 has a long and narrow rectangular shape having a length L and a width W in a plan view.

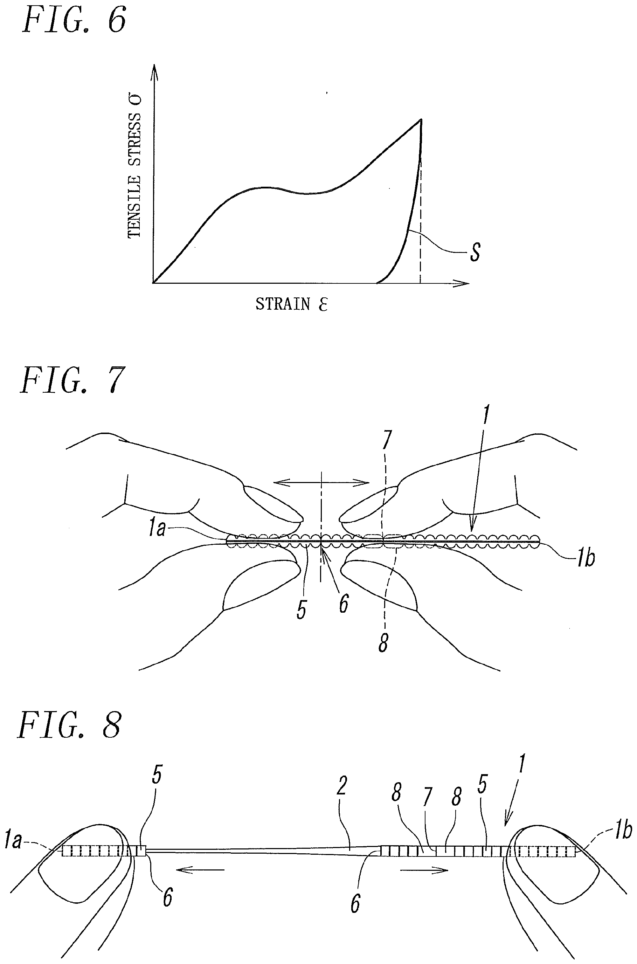

A synthetic resin easily elongated with fingers to be transformed (extended) into the plastic region and having resilient elasticity even after being extended is used as an example of the synthetic resin forming the base piece 3. Such a synthetic resin is generally classified into, among various types of synthetic resin, a synthetic resin having soft and tenacious tensile properties (mechanical characteristics) representing a "tensile stress-strain curve" illustrated in Fig. 6. Examples of synthetic resin classified into such a category include a polyolefin resin and a polyvinyl chloride resin. More preferably, polyethylene, or particularly among various types of polyethylene, low density polyethylene is most suitably used. Desirably, the base piece 3 is formed from the above synthetic resin, having its axis not extended or extended with an extremely low ratio in, particularly, the longitudinal direction during film manufacture.

The graph illustrated in Fig. 6 represents the relationship between the tensile stress and the strain when the tensile force is exerted on the synthetic resin to extend the synthetic resin and the tensile force is unloaded before the material is broken. In Fig. 6, the curved portion denoted with s after the tensile force is unloaded represents the resilient elasticity after the synthetic resin is extended according to the present invention, that is, the elastic contractility exerted on the eyelid skin. The synthetic resin having the tensile property as illustrated in Fig. 6 forms a necking when extended into the plastic region. This necking is generally transmitted gradually in the tensile direction with the progress of the extension.

As to the dimensions of the base piece 3, when the base piece 3 is formed from low density polyethylene, the length L is to be determined in consideration of, for example, the operability or portability in practical use, but is preferably approximately 25 to 40 mm. The width W and a thickness t1 of the base piece 3 are to be determined in consideration of, for example, the width or thickness of the base piece 3 extended and bonded to an eyelid and the user's feelings when the base piece 3 is attached to the eyelid. Preferably, however, the width W is approximately 1.0 to 5.0 mm, and the thickness t1 is approximately 40 to 80 µm.

The dimensions of the base piece 3 are not limited to these ranges, and may be determined appropriately in consideration of, for example, the material of the base piece 3 and the sensory evaluation, such as the operability, the user's feelings, and the appearance of the formed double eyelid.

An acrylic pressure sensitive adhesive for the skin is preferably used as an example of an adhesive forming the adhesive layers 4. However, the adhesive is not limited to this, and may be any adhesive usable for the skin. In consideration of, for example, the adhesiveness to the eyelid skin, preferably, the thickness t2 of the adhesive layers 4 is approximately 40 to 60 µm. However, the thickness is not limited to fall within this range and may be appropriately determined in accordance with the characteristics of the adhesive. The adhesive layers 4 on both surfaces of the tape-shaped member 2 may be formed from different adhesives, and may have different thicknesses t2.

The double-eyelid-forming tape 1 is formed from a synthetic resin that has releasability from the adhesive layers 4. The double-eyelid-forming tape 1 has a pair of release sheet pieces 5, bonded to the surfaces (surfaces to be bonded to the eyelid) of the adhesive layers 4. These release sheet pieces 5 have the same shape and the same dimensions as the tape-shaped member 2, that is, the base piece 3 in a plan view to cover the entirety of the surfaces (bonded surfaces) of the adhesive layers 4 of the tape-shaped member 2. These release sheet pieces 5 each have tearable portions 6 and 7, which are easily tearable by being pulled in the longitudinal direction.

Here, the tearable portions 6 and 7 include first tearable portions 6 and a second tearable portion 7. The first tearable portions 6 are formed at positions deviating from a center O of the tape 1 in the direction of the axis 1 toward a first end 1a and a second end 1b, which are both ends in the direction of the axis 1. The second tearable portion 7 is formed at the center O. These tearable portions 6 and 7 are open to the outer surfaces of the release sheet pieces 5, opposite to the inner surfaces bonded to the adhesive layers 4, and respectively have shapes of grooves having depths d1 and d2 to the middle of the thickness t3 of the release sheet pieces 5 and extending throughout the release sheet piece 5 in the width direction. Here, the depths d1 and d2 of the tearable portions 6 and 7 are different from each other, and larger than half the thickness t3 of the release sheet pieces 5. These first and second tearable portions 6 and 7 are formed to face each other to form pairs at the same positions of the pair of release sheet pieces 5 in the direction of the axis 1. The release sheet pieces 5 have inner surfaces that are smooth, mirror-like flat surfaces, and outer surfaces that are rough surfaces.

More specifically, each of the first tearable portions 6 is formed to have its groove width gradually expanding from its groove bottom 61 toward the outer surface of the corresponding release sheet piece 5, and the groove bottom 61 is formed in a flat surface parallel to the inner surface. The multiple first tearable portions 6 having the same shape and size are formed at positions symmetric with respect to the center O on both sides of the release sheet piece 5 closer to the first end 1a and the second end 1b, and arranged at a regular pitch (gap) p in the direction of the axis 1. Here, the groove bottoms 61 of each adjacent pair of the first tearable portions 6 are connected to each other with a corresponding one of convex arcuate surfaces 62 having a height d1 from the groove bottom 61. Opposing side walls of each first tearable portion 6 are thus formed by the arcuate surfaces 62 curving toward the groove inner sides.

The second tearable portion 7 is also formed to have its groove width gradually expanding from its groove bottom 71 toward the outer surface of the corresponding release sheet piece 5. As described above, the second tearable portions 7 are formed at the center O of the pair of release sheet pieces 5 in the direction of the axis 1 to oppose each other. Each second tearable portion 7 has a groove bottom 71 having an approximately acute angle and a depth d2, which is larger than the depth d1 of the first tearable portions 6. Opposing side walls of each second tearable portion 7 are thus formed by arcuate surfaces 72 curving toward the groove inner sides. Between the second tearable portion 7 and the first tearable portion 6 on each side of or adjacent to the second tearable portion 7, a flat portion 8 having a length in the direction of the axis 1 equivalent to the pitch p of the first tearable portion 6 and parallel to the groove bottoms 61 of the first tearable portions 6 is disposed. Both ends of the flat portion 8 in the direction of the axis l are smoothly connected to the arcuate surface 62 of the first tearable portion 6 and the arcuate surface 72 of the second tearable portion 7. The distance from the inner surface of the release sheet piece 5 bonded to the adhesive layer 4 to the top of the arcuate surface 62 is equal to the distance from the inner surface to the flat portion 8. This distance serves as the thickness t3 of the release sheet piece 5.

Here, each release sheet piece 5 may be an integrated unit formed from a material having tearableness at the tearable portions 6 and 7 and the releasability from the adhesive. Preferably, the release sheet piece 5 is an integrated unit formed from a silicone resin. A silicone resin having a higher hardness is preferable, because it has higher tearableness. The thickness t3 is desirably approximately 0.5 to 0.6 mm, but is not particularly limited to this. Desirably, the pitch p of the first tearable portions 6 is approximately 1.0 mm, the depth d1 of the first tearable portions 6 is approximately 65 to 70% of the thickness t3 of the release sheet pieces 5, the depth d2 of the second tearable portion 7 is approximately 85 to 90% of the thickness t3 of the release sheet pieces 5, the width e of the flat groove bottoms 61 of the first tearable portions 6 is approximately 8% to 10% of the pitch p, and the angle of the groove bottom 71 of the second tearable portion 7 is approximately 40 degrees. These properties, however, are not particularly limitative to the above values.

A method for forming a double eyelid using the double-eyelid-forming tape 1, serving as a cosmetic eyelid shaping tape according to the present invention, is described below with reference to Fig. 7 to Fig. 13. Here, an example is described where a first tearable portion 6 deviating from the center O toward the first end 1a is to be torn.

Although not illustrated, oil and other matter are wiped off an upper eyelid 90 to be formed into a double eyelid, and then the position at which the double eyelid fold 92 is to be formed is checked using a device such as a pusher. Subsequently, as illustrated in Fig. 7, the double-eyelid-forming tape 1 is pinched between fingers over the pair of release sheet pieces 5 of the double-eyelid-forming tape 1 on both sides of the first tearable portions 6 of the release sheet pieces 5 located at the positions intended to be torn. Then, the release sheet pieces 5 are torn at the first tearable portions 6.

Here, as illustrated in Fig. 7, to prevent the release sheet pieces 5 from being torn at the second tearable portions 7, the second tearable portions 7 are desirably held with fingers. At this time, the flat portions 8 formed on both sides of the second tearable portions 7 serve as guides. To tear the release sheet pieces 5 at the intended positions as accurately as possible, the distances from the first tearable portions 6 at the intended to-be-torn positions to the fingers on both sides are desirably as equal to each other and short as possible.

When the release sheet pieces 5 are thus torn, between fingers, the release sheet pieces 5 are released from the adhesive layers 4 of the tape-shaped member 2, and concurrently, the tape-shaped member 2 is extended and partially exposed. Normally, the base piece 3 of the corresponding portion is accordingly transformed into the plastic region and extended. When the base piece 3 has a wide elastic region, desirably, the tape-shaped member 2 is fully elongated so that the base piece 3 at the corresponding portion can be extended.

Subsequently, both end portions of the tape-shaped member 2 are pinched between fingers over the release sheet pieces 5 torn at the first tearable portions 6. Here, as illustrated in Fig. 8, the lengths from the first end 1a and the second end 1b to the pinched portions are desirably substantially equal to each other. At this time, the rows of the first tearable portions 6 also serve as nonslip portions for fingers that pinch the release sheet pieces 5.

As illustrated in Fig. 8, when the portions of the tape-shaped member 2 pinched between fingers on both sides are elongated to at least a length necessary for forming a double eyelid, the extension of the base piece 3 is usually transmitted gradually in the direction of the axis 1 from the position near the torn first tearable portions 6 since the base piece 3 has a uniform width throughout in the direction of the axis 1. At this time, the adhesive layers 4 are also gradually thinned in accordance with the progress of the extension of the base piece 3. However, the transmission and the progress of extension are slower toward the second end 1b, as the distance from the torn first tearable portions 6 becomes longer. Thus, the adhesive layers 4 at portions closer to the second end 1b and having a smaller extension ratio are left thicker, and secure more efficient adhesiveness.

On the other hand, within predetermined ranges from near the torn tearable portion to the first end 1a and the second end 1b, the base piece 3 is fully extended to have sufficiently large resilient elasticity after the extension.

By appropriately adjusting the relationship between the releasability of the release sheet pieces 5 from the adhesive layers 4 and the extensibility of the base piece 3, the above-described efficient adhesiveness and sufficiently large resilient elasticity can be secured by merely tearing the first tearable portions 6 and elongating the tape-shaped member 2 to a necessary length, as illustrated in Fig. 8.

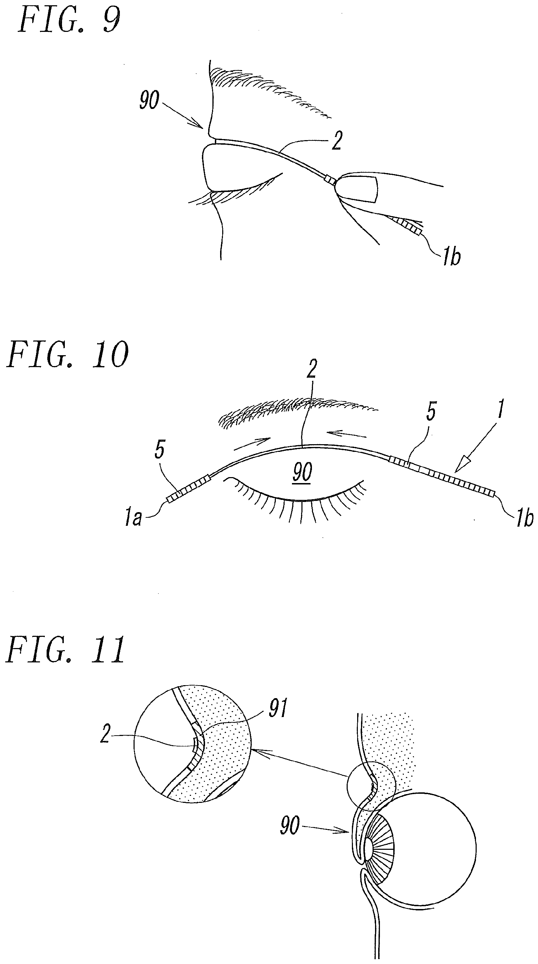

Subsequently, while being pulled tightly, the tape-shaped member 2 thus elongated to a necessary length is pressed against the portion (line) of the upper eyelid 90 at which the double eyelid fold 92 is intended to be formed, as illustrated in Fig. 9, to be bonded to the upper eyelid 90 with the adhesive layers 4. Subsequently, as illustrated in Fig. 10, both hands let go of the tape 1 to unload the tensile force exerted on the tape-shaped member 2 and the pressing force exerted on the upper eyelid 90. Thus, as illustrated in Fig. 11, the elastic contractile force of the extended base piece 3 is exerted on the upper eyelid 90, and the tape-shaped member 2 (that is, the base piece 3) is introduced into the upper eyelid 90 to form a constricted portion 91 in the upper eyelid 90.

Here, as described above, the portion of the tape-shaped member 2 near the outer canthus, that is, the portion near the second end 1b having a smaller extension ratio has thicker portions of the adhesive layers 4. Thus, the portion has more efficient adhesiveness and can thus be more securely bonded to the skin of the upper eyelid 90. Thus, when the base piece 3 elastically contracts, the tape-shaped member 2 and the skin of the upper eyelid 90 are prevented from slipping over each other. Thus, a constriction corresponding to the actual amount of contraction of the tape-shaped member 2, that is, a constricted portion 91 having a depth necessary for forming an imitation double eyelid fold 92 can be more reliably formed in the upper eyelid 90.

In this manner, in the state where the constricted portion 91 is formed in the upper eyelid 90 with the elastic contractility after extension of the base piece 3 of the tape-shaped member 2, the tape-shaped member 2 is bonded to the bottom of the constricted portion 91, as illustrated in Fig. 11.

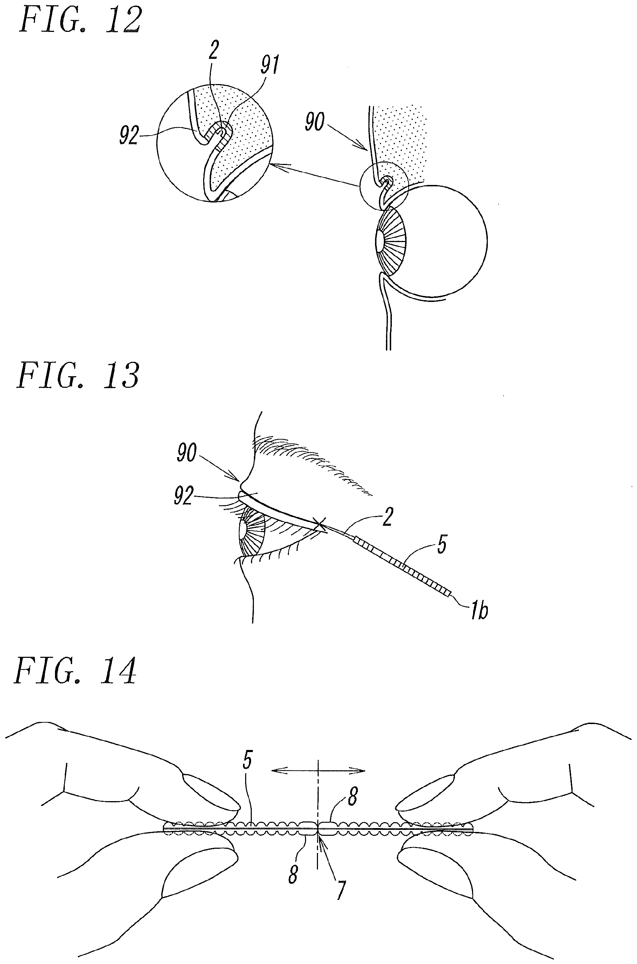

As illustrated in Fig. 12 and Fig. 13, when the upper eyelid 90 is raised to open the eye, the upper eyelid 10 is naturally folded along the constricted portion 91 to form the double eyelid fold 92.

An unnecessary extra portion of the tape-shaped member 2 bonded to the upper eyelid 90 may be cut off with, for example, scissors or a cutter at an appropriate position (position denoted with X in Fig. 13). Thereafter, the shape of the double eyelid may be adjusted with, for example, a pusher, not illustrated.

Furthermore, as in the double-eyelid-forming tape disclosed in, for example, Japanese Patent No. 3277180, the double-eyelid-forming tape 1 may be used by tearing the release sheet pieces 5 at the center O. In this case, as illustrated in Fig. 14, the tape 1 is pinched between fingers on both ends 1a and 1b of the tape 1 while having necessary distances apart from the center O of the tape 1. Then, the tape 1 is pulled in the direction of the axis 1 to tear the release sheet pieces 5 at the second tearable portions 7 at the center O. Here, the second tearable portions 7 are grooves deeper than the first tearable portions 6, and have their groove bottoms 71 having substantially acute angles. Thus, the second tearable portions 7 are more easily tearable than the first tearable portions. Thus, the second tearable portions 7 can be torn prior to the first tearable portions 6 when the tape 1 is pulled in both directions of the axis 1. As described above, the flat portions 8 are formed on both sides of the second tearable portion 7. Thus, the flat portions 8 can be used as the guides for pinching or handling the tape 1 to easily visually check the position of the second tearable portion 7.

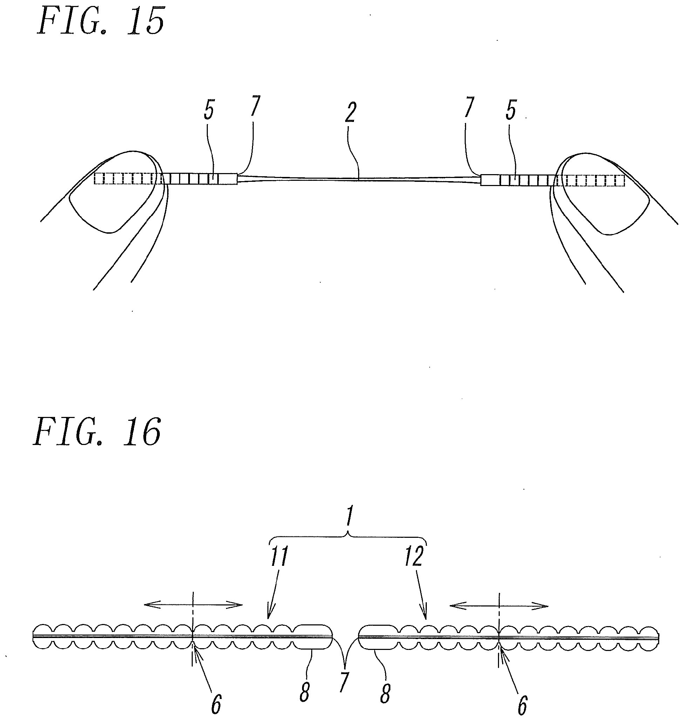

As illustrated in Fig. 15, when the tape-shaped member 2 is elongated to a necessary length as it is, the base piece 3 can be extended into the plastic region substantially laterally equidistantly (that is, laterally symmetrically) from the center O. Specifically, when the tape-shaped member 2 is elongated to a necessary length, the thickness of the adhesive layers 4 can be made laterally equal to each other (that is, laterally symmetrically).

Thus, in the case where the release sheets 5 are thus torn at the second tearable portions 7, well-balanced adhesiveness to the eyelid skin and resilient elasticity after extension can be secured on the sides of the tape-shaped member 2 closer to the first end 1a and the second end 1b.

The operations after Fig. 15 are substantially the same as that in the case where the tape is torn at the first tearable portions 6 (Fig. 9 to Fig. 13), and thus are not described to prevent redundancy.

The double-eyelid-forming tape 1, serving as a cosmetic eyelid shaping tape according to the present invention, includes the release sheet pieces 5 each having the first tearable portions 6 and the second tearable portion 7. Thus, as described above, besides improving the adhesiveness of the tape-shaped member 2 at portions closer to the inner canthus and the outer canthus, and preventing fingers from slipping when elongating the tape-shaped member 2, the double-eyelid-forming tape 1 can change the extension ratio of the base piece 3 in the direction of the axis 1 (that is, change the elastic contractility after extension in the direction of the axis 1) by adjusting the torn positions of the release sheet pieces 5 or the elongation length of the tape-shaped member to correspond to the states of the individual eyelids 90 or the preference of the outcome of the double eyelid. In addition, by employing these tearable portions 6 and 7 as part of the design of the tape 1, the design quality of the tape 1 can be also improved.

Fig. 16 illustrates another example of a method for using the double-eyelid-forming tape 1. In this method, first, the tape 1 is separated at the center O, that is, the release sheet pieces 5 are torn at the second tearable portions 7, and the tape-shaped member 2 is cut at the same position to be divided into a first tape 11 and a second tape 12. Here, the second tearable portion 7 can be torn more easily than the first tearable portions 6. Thus, for example, by firmly pulling the tape 1 in both directions of the axis 1 while pinching portions of the second tearable portion 7 near both sides, the tape 1 can be easily divided into the first tape 11 and the second tape 12.

Subsequently, the release sheet pieces of the tapes 11 and 12 are torn at appropriate first tearable portions 6, and the respective tape-shaped members 2 are elongated to extend the respective base pieces 3 into the plastic region. Then, with the method described above with reference to Fig. 9 to Fig. 13, the tapes 11 and 12 are respectively applied to, for example, left and right upper eyelids 90 to form double eyelids on the left and right upper eyelids 90 with a single double-eyelid-forming tape 1.

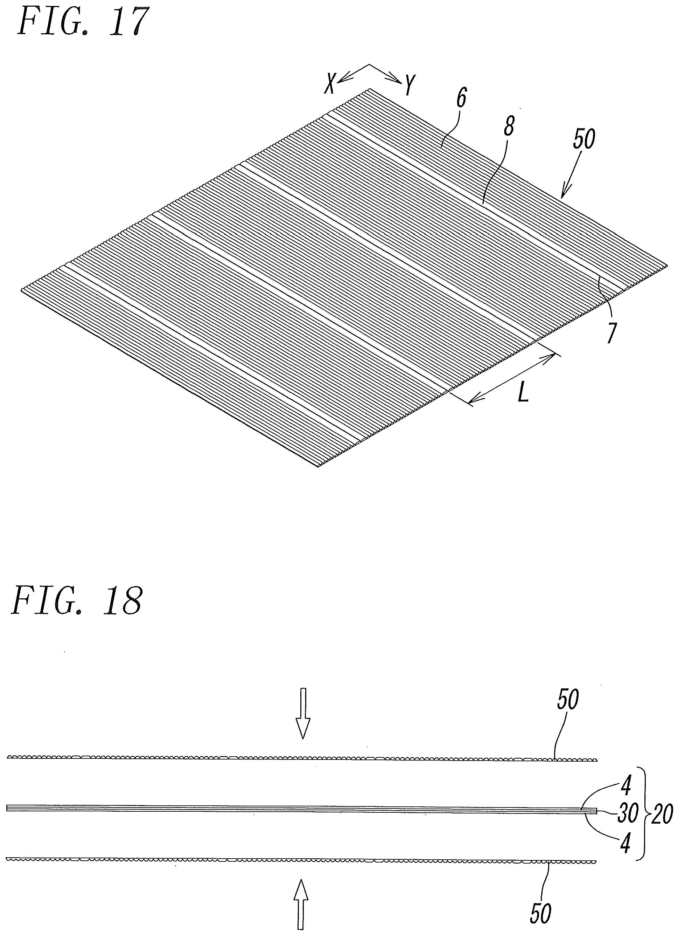

Subsequently, a method for manufacturing the above double-eyelid-forming tape 1, that is, a cosmetic eyelid shaping tape according to the present invention is described with reference to Fig. 17 to Fig. 20.

First, a double-sided adhesive sheet 20, which includes a base film (such as a low density polyethylene film) 30 forming the base piece 3 and an adhesive layers 4 on the entire top and bottom surfaces of the base film 30, are prepared (Fig. 18).

On the other hand, as illustrated in Fig. 17, a silicone resin having high hardness is thermoformed to form release sheets 50 having a thickness t3 for forming the release sheet pieces 5. Here, each inner surface, which is to be bonded to the adhesive layers 4, is formed into a mirror-like surface, and each outer surface, opposite to the inner surface, is formed into a rough surface. Concurrently, the first tearable portions 6, the second tearable portions 7, and the flat portions 8 are formed on each outer surface to extend in a Y direction parallel to each other. Here, to obtain multiple release sheet pieces 5 in the X direction, multiple combinations each including one second tearable portion 7 and a pair of flat portions 8 on both sides are arranged in the X direction at the interval the same as the length L of the double-eyelid-forming tape. Between each adjacent pair of the combinations, the first tearable portions 6 are formed throughout at a regular pitch p. Thus, the first and second tearable portions 6 and 7 are concurrently formed when integrally forming the release sheet 50. Thus, the release sheet 50 can be formed without making the process for manufacturing the multiple tearable portions 6 and 7 complex.

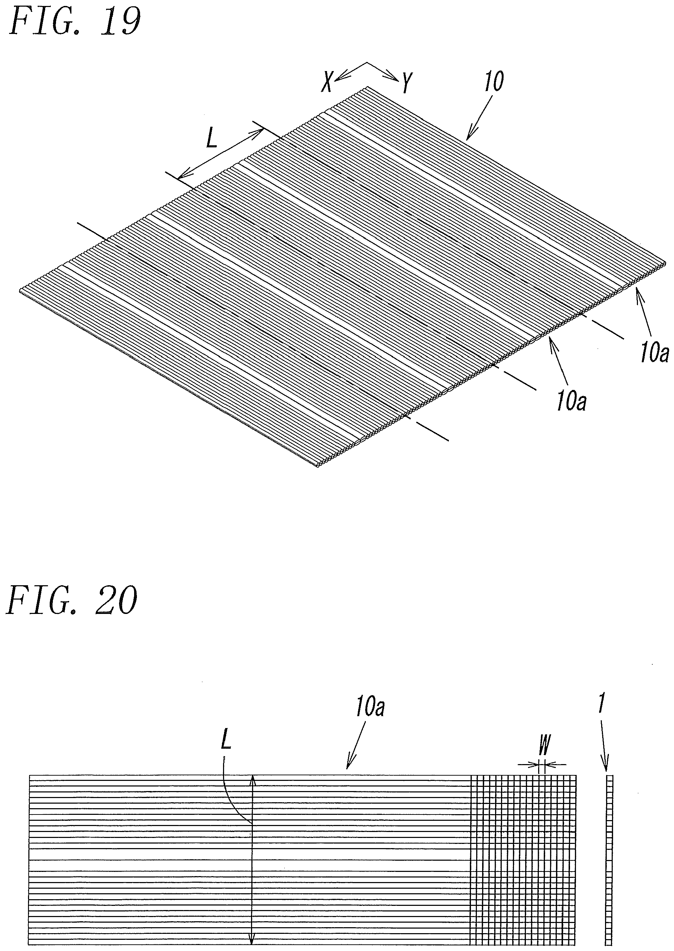

As illustrated in Fig. 18, two release sheets 50 and a double-sided adhesive sheet 20 are opposed to and bonded to each other while having the flat inner surfaces of the release sheets 50 facing the adhesive layers 4 of the double-sided adhesive sheet 20 to thus form a laminate sheet 10, illustrated in Fig. 19. Here, the tearable portions 6 and 7 of the two release sheets 50 are disposed parallel to and opposite to each other.

When the base film 30 is a polyolefin resin film, desirably, the film does not extend or extends with an extremely low ratio in the width direction, perpendicular to the direction of transport during manufacture, and the release sheets 50 and the double-sided adhesive sheet 20 are bonded together so that the width direction of the film is perpendicular to the tearable portions 6 and 7 of the release sheets 50 (or coincides with the X direction).

Subsequently, as illustrated in Fig. 19, by cutting the laminate sheet 10 parallel to the tearable portions 7 (or in the Y direction) at the middle of the second tearable portions 7 adjacent to each other in the X direction, long and narrow strip-shaped laminate sheets 10a, as illustrated in Fig. 20, are formed. Then, as illustrated in Fig. 20, the strip-shaped laminate sheets 10a are cut at regular intervals W by a cutting blade (not illustrated) extending in the direction perpendicular to the tearable portions 6 and 7. Thus, the double-eyelid-forming tapes 1 each having the second tearable portion 7 at the middle in the longitudinal direction (the direction of the axis 1) can be obtained.

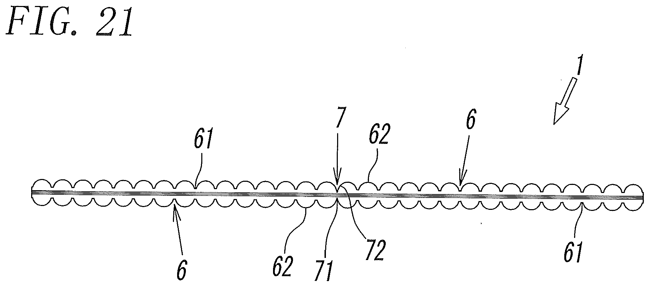

The form of the release sheet pieces 5 is not limited to the above-described form. For example, as illustrated in Fig. 21, without forming the flat portions 8 on both sides of the second tearable portion 7 at the middle, all the groove bottoms 61 and 71 of the adjacent first and second tearable portions 6 and 7 may be connected with the arcuate surfaces 62 and 72. The first tearable portions 6 may be formed on either one of the sides of the center O closer to the first end 1a and the second end 1b. The second tearable portions 7 are not necessarily provided. The number or the pitch p of the first tearable portions 6 is not particularly limited to that of the embodiment. The tearable portions 6 and 7 may have a form of a letter V-shaped cross section (V notch) having flat side walls.

In the above description, the double-eyelid-forming tape 1 is described as an example of an embodiment of a cosmetic eyelid shaping tape according to the present invention. However, the present invention is also applicable to, for example, a lower eyelid tape for forming puffiness (a so-called eye bag) at a lower eyelid. In this case, as in the case of forming the above-described double eyelid, the tape-shaped member 2 is bonded to a lower eyelid while being elongated to a predetermined length for which the base piece 3 is extended, the tape-shaped member 2 is introduced into the lower eyelid with the elastic contractility after extension of the base piece 3. Thus, a constricted portion extending along the tape-shaped member 2 is formed at the portion. Thus, puffiness (eye bag) is formed above the constricted portion formed in the lower eyelid, creating an eye with depth and height. To apply the present invention to such a lower eyelid tape, the length L of the tape may be made shorter than that of the double-eyelid-forming tape 1. As to other portions, the components are basically the same as those of the above-described double-eyelid-forming tape 1, and thus are not described to prevent redundancy.

Thus far, embodiments of the present invention have been described in detail. However, the present invention is not limited to these, and may naturally be changed in design in various manners within the scope not departing from the gist of the present invention. For example, within the scope not impeding the extension operation of the base piece 3 or the resilient elasticity after the extension, another base piece (such as a polyurethane resin film) may be laminated on the base piece 3 to form a tape-shaped member 2.

### Reference Signs List

- 1: cosmetic eyelid shaping tape (double-eyelid-forming tape)
- 1a: first end
- 1b: second end
- 2: tape-shaped member
- 3: base piece
- 4: adhesive layer
- 5: release sheet piece
- 6: first tearable portion
- 7: second tearable portion
- 8: flat portion
- 10: laminate sheet
- 10a: strip-shaped laminate sheet
- 11: first tape
- 12: second tape
- 20: double-sided adhesive sheet
- 30: base film
- 50: release sheet
- 61, 71: groove bottom
- 62, 72: arcuate surface
- 90: upper eyelid
- 91: constricted portion
- 92: double eyelid fold
- d1: depth of first tearable portion 6
- d2: depth of second tearable portion 7
- e: width of groove bottom 61
- 1: axis
- L: length
- O: center
- p: pitch of first tearable portion 6
- t1: thickness of base piece 3
- t2: thickness of adhesive layer 4
- t3: thickness of release sheet piece 5
- W: width

## Claims

1. A cosmetic eyelid shaping tape for performing pseudo plastic surgery on an eyelid by forming a constricted portion in the eyelid, the tape comprising: a tape-shaped member including a long and narrow base piece having a uniform width and adhesive layers laminated on both surfaces of the base piece, the base piece being formed from a synthetic resin extensive into a plastic region and having resilient elasticity even after being extended, the constricted portion being formed in the eyelid using elastic contractility of the base piece when the tape-shaped member is elongated until the base piece is extended,
wherein the cosmetic eyelid shaping tape further comprises a pair of release sheet pieces formed from a synthetic resin that is easily releasable from the adhesive layers, the release sheet pieces being bonded to cover the adhesive layers of the tape-shaped member, the release sheet pieces each having a first end and a second end on both ends in a longitudinal direction,
wherein the pair of release sheet pieces each have a tearable portion on at least one side of a longitudinal center closer to the first end and/or the second end, the tearable portion being torn when being pulled in the longitudinal direction, and
wherein the tearable portion has a shape of a groove having a depth from an outer surface of each release sheet piece to a middle in a thickness direction and extending in a width direction.

2. The cosmetic eyelid shaping tape according to Claim 1,
wherein the tearable portion has a shape of a groove having a groove width gradually expanding from a groove bottom toward the outer surface of the release sheet piece.

3. The cosmetic eyelid shaping tape according to Claim 2,
wherein the groove bottom of the tearable portion forms a flat surface.

4. The cosmetic eyelid shaping tape according to either one of Claim 2,
wherein a plurality of the tearable portions are formed at a regular pitch in the longitudinal direction of the release sheet pieces.

5. The cosmetic eyelid shaping tape according to Claim 4,
wherein the groove bottoms of the tearable portions adjacent to each other are connected together with arcuate surfaces rising from the groove bottoms and having the same height.

6. The cosmetic eyelid shaping tape according to Claim 1,
wherein each of the release sheet pieces has the same number of tearable portions on both sides of the longitudinal center closer to the first end and the second end.

7. The cosmetic eyelid shaping tape according to Claim 6,
wherein each of the release sheet pieces has the tearable portions at symmetrical positions on the sides closer to the first end and the second end with respect to the longitudinal center.

8. The cosmetic eyelid shaping tape according to Claim 1,
wherein the tearable portion forms a first tearable portion, and the pair of release sheet pieces each further include a second tearable portion at the longitudinal center, the second tearable portion being torn when being pulled in the longitudinal direction, and
wherein the second tearable portion has a shape of a groove having a depth from an outer surface of each release sheet piece to a middle in a thickness direction and extending in a width direction.

9. The cosmetic eyelid shaping tape according to Claim 8,
wherein the second tearable portion has a shape of a groove having a groove width gradually expanding from a groove bottom toward the outer surface of the release sheet piece, and having a larger depth than the first tearable portion.

10. The cosmetic eyelid shaping tape according to Claim 9,
wherein the groove bottom of the second tearable portion forms an acute angle.

11. The cosmetic eyelid shaping tape according to any one of Claims 8 to 10,
wherein the pair of release sheet pieces each have a plurality of the first tearable portions on both sides of the second tearable portion in the longitudinal direction closer to the first end and the second end, the first tearable portions being arranged at a regular pitch in the longitudinal direction of the release sheet piece, and
wherein, on an outer surface of the release sheet piece at a portion adjacent to the second tearable portion in the longitudinal direction, a flat portion not including the first tearable portion is formed.

## Amended claims

### Amended claims under Art. 19.1 PCT

1. (Amended) A cosmetic eyelid shaping tape for performing pseudo plastic surgery on an eyelid by forming a constricted portion in the eyelid, the tape comprising: a tape-shaped member including a long and narrow base piece having a uniform width and adhesive layers laminated on both surfaces of the base piece, the base piece being formed from a synthetic resin extensive into a plastic region and having resilient elasticity even after being extended, the constricted portion being formed in the eyelid using elastic contractility of the base piece when the tape-shaped member is elongated until the base piece is extended,
wherein the cosmetic eyelid shaping tape further comprises a pair of release sheet pieces formed from a synthetic resin that is easily releasable from the adhesive layers, the release sheet pieces being bonded to cover the adhesive layers of the tape-shaped member, the release sheet pieces each having a first end and a second end on both ends in a longitudinal direction,
wherein the pair of release sheet pieces each have a tearable portion on at least one side of a longitudinal center closer to the first end and/or the second end, the tearable portion being torn when being pulled in the longitudinal direction, and
wherein the tearable portion has a shape of a groove having a depth from an outer surface of each release sheet piece to a middle in a thickness direction and extending in a width direction, the groove having a groove width gradually expanding from a groove bottom toward the outer surface of the release sheet piece, and the groove bottom of the tearable portion forming a flat surface.

2. (Amended) The cosmetic eyelid shaping tape according to Claim 1,
wherein each of the release sheet pieces has the same number of tearable portions on both sides of the longitudinal center closer to the first end and the second end.

3. (Amended) The cosmetic eyelid shaping tape according to Claim 2,
wherein each of the release sheet pieces has the tearable portions at symmetrical positions on the sides closer to the first end and the second end with respect to the longitudinal center.

4. (Amended) A cosmetic eyelid shaping tape for performing pseudo plastic surgery on an eyelid by forming a constricted portion in the eyelid, the tape comprising: a tape-shaped member including a long and narrow base piece having a uniform width and adhesive layers laminated on both surfaces of the base piece, the base piece being formed from a synthetic resin extensive into a plastic region and having resilient elasticity even after being extended, the constricted portion being formed in the eyelid using elastic contractility of the base piece when the tape-shaped member is elongated until the base piece is extended,
wherein the cosmetic eyelid shaping tape further comprises a pair of release sheet pieces formed from a synthetic resin that is easily releasable from the adhesive layers, the release sheet pieces being bonded to cover the adhesive layers of the tape-shaped member, the release sheet pieces each having a first end and a second end on both ends in a longitudinal direction,
wherein the pair of release sheet pieces each have a tearable portion on at least one side of a longitudinal center closer to the first end and/or the second end, the tearable portion being torn when being pulled in the longitudinal direction, and
wherein the tearable portion has a shape of a groove having a depth from an outer surface of each release sheet piece to a middle in a thickness direction and extending in a width direction, the groove having a groove width gradually expanding from a groove bottom toward the outer surface of the release sheet piece, and
wherein a plurality of the tearable portions are arranged at a regular pitch in the longitudinal direction of the release sheet piece.

5. The cosmetic eyelid shaping tape according to Claim 4,
wherein the groove bottoms of the tearable portions adjacent to each other are connected together with arcuate surfaces rising from the groove bottoms and having the same height.

6. (Amended) The cosmetic eyelid shaping tape according to Claim 4,
wherein each of the release sheet pieces has the same number of tearable portions on both sides of the longitudinal center closer to the first end and the second end.

7. The cosmetic eyelid shaping tape according to Claim 6,
wherein each of the release sheet pieces has the tearable portions at symmetrical positions on the sides closer to the first end and the second end with respect to the longitudinal center.

8. (Amended) A cosmetic eyelid shaping tape for performing pseudo plastic surgery on an eyelid by forming a constricted portion in the eyelid, the tape comprising: a tape-shaped member including a long and narrow base piece having a uniform width and adhesive layers laminated on both surfaces of the base piece, the base piece being formed from a synthetic resin extensive into a plastic region and having resilient elasticity even after being extended, the constricted portion being formed in the eyelid using elastic contractility of the base piece when the tape-shaped member is elongated until the base piece is extended,
wherein the cosmetic eyelid shaping tape further comprises a pair of release sheet pieces formed from a synthetic resin that is easily releasable from the adhesive layers, the release sheet pieces being bonded to cover the adhesive layers of the tape-shaped member, the release sheet pieces each having a first end and a second end on both ends in a longitudinal direction,
wherein the pair of release sheet pieces each have first tearable portions on a side of a longitudinal center closer to the first end and/or the second end, and a second tearable portion at the longitudinal center, the first tearable portions being torn when being pulled in the longitudinal direction, and the second tearable portion being torn when being pulled in the longitudinal direction,
wherein the first and second tearable portions each have a shape of a groove having a depth from an outer surface of each release sheet piece to a middle in a thickness direction and extending in a width direction, the groove having a groove width gradually expanding from a groove bottom toward the outer surface of the release sheet piece, and
wherein the second tearable portion is deeper than the first tearable portions.

9. (Amended) The cosmetic eyelid shaping tape according to Claim 8,
wherein the groove bottom of each of the first tearable portions forms a flat surface, and the groove bottom of the second tearable portion forms an acute angle.

10. (Amended) The cosmetic eyelid shaping tape according to Claim 8,
wherein the pair of release sheet pieces each have a plurality of the first tearable portions on both sides of the second tearable portion in the longitudinal direction closer to the first end and the second end, the first tearable portions being arranged at a regular pitch in the longitudinal direction of the release sheet piece.

11. (Amended) The cosmetic eyelid shaping tape according to Claim 10,
wherein the groove bottoms of the first tearable portions adjacent to each other are connected together with arcuate surfaces rising from the groove bottoms and having the same height, and
wherein flat portions formed of flat surfaces are formed at portions on an outer surface located between the second tearable portion and a pair of the first tearable portions adjacent to the second tearable portion, and a thickness of each release sheet piece at a top portion of the arcuate surface is equal to a thickness of each release sheet piece at the flat portions.

Statement under Art. 19.1 PCT
Claim 1 is amended by adding Claim 2 and Claim 3, which is said to have novelty and inventive step, to Claim 1 in the application as originally filed to clarify the point that the groove bottom of the groove-shaped tearable portion forms a flat surface. This point is disclosed in none of the cited documents. Claims 2 and 3, directly or indirectly citing Claim 1, thus have novelty and inventive step.

Claim 4 is amended as an independent claim based on Claim 4 in the application as originally filed, which is said to have novelty and inventive step. The point that a plurality of tearable portions are arranged at a regular pitch in the longitudinal direction of each release sheet piece is disclosed in none of the cited documents. Claims 5 to 7, directly or indirectly citing Claim 4, thus have novelty and inventive step.

Claim 8 is amended as an independent claim based on Claim 9 in the application as originally filed, which is said to have novelty and inventive step, and by adding Claim 2 in the application as originally filed. The point that the second tearable portion has a shape of a groove having a groove width gradually expanding from a groove bottom toward the outer surface of the release sheet piece, and having a larger depth than the first tearable portion is disclosed in none of the cited documents. Claims 9 to 11, directly or indirectly citing Claim 8, thus have novelty and inventive step.
